# FASCICULE DE BREVET EUROPEEN

(11) **EP 0 966 260 B1**
(45) Date de publication et mention de la délivrance du brevet: **28.05.2003**
(21) Numéro de dépôt: 98955651.9
(22) Date de dépôt: 13.11.1998
(51) Int. Cl.: A61K 7/42, A61K 7/48

(54) **EMULSIONS HUILE-DANS-EAU CONTENANT UN DERIVE DE 1,3,5-TRIAZINE ET UNE SILICONE COPOLYOL ET APPLICATIONS COSMETIQUES**
ÖL-IN-WASSER EMULSIONEN ENTHALTEND EIN 1,3,5 TRIAZINDERIVAT UND EIN SILIKONCOPOLYOL UND KOSMETISCHE VERWENDUNGEN
OIL-IN-WATER EMULSIONS CONTAINING A 1,3,5-TRIAZINE DERIVATIVE AND A COPOLYOL SILICONE AND COSMETIC APPLICATIONS

(30) Priorité: 04.12.1997 FR 9715310
(43) Date de publication de la demande: 29.12.1999
(73) Titulaire: L'OREAL, 75008 Paris (FR)
(72) Inventeur: HANSENNE, Isabelle, F-75017 Paris (FR); JOSSO, Martin, F-75005 Paris (FR); NODARI, Laurent, F-95100 Argenteuil (FR)
(74) Mandataire: Miszputen, Laurent
(86) Numéro de dépôt international: FR9802425
(87) Numéro de publication internationale: WO99029291

(56) Documents cités:
- JP-A- 7 033 628

## Description

La présente invention a trait de nouvelles émulsions huile-dans-eau comprenant i) au moins un dérivé de 1,3,5-triazine particulier et ii) au moins un polyalkyl polyéther siloxane portant des groupes polyoxyalkylénés greffés sur la chaîne siliconée principale ainsi qu'à leurs utilisations cosmétiques pour la photoprotection de la peau et/ou des cheveux et/ou d'autres matières kératiniques contre le rayonnement ultraviolet, en particulier le rayonnement solaire.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques et/ou dermatologiques destinées à la photoprotection (UV-A et/ou UV-B) de la peau ont été proposées à ce jour.

Ces compositions antisolaires se présentent assez souvent sous la forme d'une émulsion, de type huile-dans-eau (c'est-à-dire un support cosmétiquement et/ou dermatologiquement acceptable constitué d'une phase continue dispersante aqueuse et d'une phase discontinue dispersée huileuse) ou eau-dans-huile (phase aqueuse dispersée dans une phase huileuse continue), qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire (SPF) s'exprimant mathématiquement par le rapport du temps d'irradiation nécessaire pour atteindre le seuil érythématogène avec le filtre UV au temps nécessaire pour atteindre le seuil érythématogène sans filtre UV). Dans de telles émulsions, les filtres hydrophiles sont présents dans la phase aqueuse et les filtres lipophiles sont présents dans la phase grasse.

Les émulsions huile-dans-eau sont, d'une manière générale, plus appréciées par le consommateur que les émulsions eau-dans-huile, en raison notamment de leur toucher agréable (voisin de l'eau) et de leur présentation sous forme de lait ou de crème non gras, tandis que les émulsions eau-dans-huile laissent après application une impression de gras particulièrement désagréable pour l'utilisateur.

Cependant, les émulsions huile-dans-eau habituellement utilisées dans les applications solaires nécessitent l'utilisation d'agents tensioactifs émulsionnants huile-dans-eau tels que des esters d'acides gras polyalcoxylés, des alcools gras polyalcoxylés ou certains tensioactifs anioniques pouvant présenter un potentiel irritant pour les peaux sensibles. Les tensioactifs émulsionnants classiques utilisés, comme le rappelle l'article intitulé «Universal Oil-in-water Polyelectrolyte Emulsifiers for Advanced Cosmetic Product Formulation» Parfumerie und Kosmetik, 72. Jahrgang. Nr 11/91, émulsifient en réduisant de façon significative les énergies interfaciales huile/eau.

Pour surmonter cet inconvénient, on a proposé d'utiliser, à la place des tensioactifs émulsionnants classiques, certains agents épaississants et/ou gélifiants du fait qu'ils permettent d'améliorer la stabilité des émulsions huile/eau et qu'ils présentent une bonne innocuité vis-à-vis des peaux sensibles. On peut citer par exemple les acides polyacryliques réticulés du type CARBOPOL, les acides polyacryliques réticulés à chaîne grasse comme le PEMULEN TR1, les gommes de guar et celluloses modifiées ou non telles que la gomme de guar hydroxypropylée, la méthylhydroxyéthylcellulose et l'hydroxypropylméthyl cellulose. Cependant, la stabilité de ces émulsions huile/eau contenant ces épaississants et/ou gélifiants n'est pas encore pleinement satisfaisante.

Certains dérivés de 1,3,5-triazine sont connus en cosmétique pour leurs propriétés absorbantes des rayonnements UV et plus particulièrement des rayons UV-B. Ils sont décrits dans les demandes de brevet EP-A-0517104, EP-A-0570838 et EP-A-0796851. On connaît également la 2,4,6-tris[p-(2'-éthylhexyl-1'-oxycarbonyl)anilino]-1,3,5-triazine vendue notamment sous la dénomination commerciale de "UVINUL T 150" par la Société BASF.

On connaît également dans la demande de brevet japonaise JP 07 033 628 des compositions capillaires pour la photoprotection des cheveux contre les effets néfastes des rayons UV contenant l'association d'un dérivé de 1,3,5-triazine (ie : UVINUL T150) et d'une silicone non-volatile, insoluble dans l'eau telle qu'un polyalkyl polyéther siloxane (ie : KF6002). L'exemple 7 décrit en particulier une composition aqueuse pour le conditionnement contenant la triazine UVINUL T150, un polyalkyl polyéther siloxane (KF6002), de l'alcool cétylique et du chlorure de cétylstéaryltriméthylammonium.

Il a été découvert de manière surprenante que, contrairement à d'autres filtres UV organiques classiques habituellement utilisés dans les applications solaires, ces mêmes dérivés de 1,3,5-triazine agissaient comme agents stabilisants dans des émulsions huile-dans-eau contenant des polyalkyl polyéther siloxanes portant des groupes polyoxyalkylénés greffés sur la chaîne siliconée.

La Demanderesse a découvert que l'utilisation de ces dérivés de 1,3,5-triazine que l'on définira en détail plus loin, en association avec des polyalkyl polyéther siloxanes portant des groupes polyoxyalkylénés greffés sur la chaîne siliconée dans les émulsions huile-dans-eau telles que définies ci-dessus contenant au moins un agent gélifiant et/ou épaississant, hydrosoluble ou hydrophile, permettait d'améliorer de façon substantielle la stabilité de ces formulations, sans qu'il soit nécessaire d'utiliser des émulsionnants tensioactifs classiques.

D'autre part, la Demanderesse a constaté que les émulsions huile-dans-eau ainsi obtenues avec cette association particulière permettaient de préparer des compositions cosmétiques ou dermatologiques pour la photoprotection de la peau et/ou des cheveux et/ou d'autres matières kératiniques, ayant de bonnes propriétés absorbantes des rayons UV et pouvant contenir une large gamme de filtres solaires hydrophiles et lipophiles choisis de telle sorte à obtenir des facteurs de protection solaire élevés et une photoprotection vis-à-vis des rayons UV dans une large gamme de longueurs d'onde (UV-A et/ou UV-B)

Ainsi, conformément à l'un des objets de la présente invention, il est maintenant proposé de nouvelles émulsions huile-dans-eau qui sont essentiellement caractérisées par le fait qu'elles comprennent i) au moins un dérivé de 1,3,5-triazine particulier, ii) au moins un polyalkyl polyéther siloxane portant des groupes polyoxyalkylénés greffés sur la chaîne siliconée principale; sous réserve que la dite émulsion ne contient pas de chlorure de cétylstéaryltriméthylammonium.

La présente invention a également pour objet l'utilisation de telles émulsions pour la fabrication de compositions cosmétiques ou dermatologiques destinées à la protection de la peau et/ou des cheveux et/ou les autres matières kératiniques humaines telles que les cils, les sourcils ou les ongles contre le rayonnement ultraviolet, en particulier le rayonnement solaire.

Un autre objet encore de la présente invention réside dans un procédé de traitement cosmétique pour la protection de la peau et/ou des cheveux et/ou les autres matières kératiniques humaines contre le rayonnement ultraviolet, en particulier le rayonnement solaire, et qui consiste essentiellement à appliquer sur ces derniers une quantité efficace d'une composition conforme à l'invention.

La présente invention a également pour objet l'utilisation des dérivés de triazine de l'invention comme agent stabilisant pour la préparation d'une émulsion huile-dans-eau contenant au moins un polyalkyl polyéther siloxane portant des groupes polyoxyalkylénés greffés sur la chaîne siliconée.

D'autres caractéristiques, aspects et avantages de la présente invention apparaîtront à la lecture de la description détaillée qui va suivre.

Les dérivés de 1,3,5-triazine conformes à l'invention répondent à la formule générale suivante : 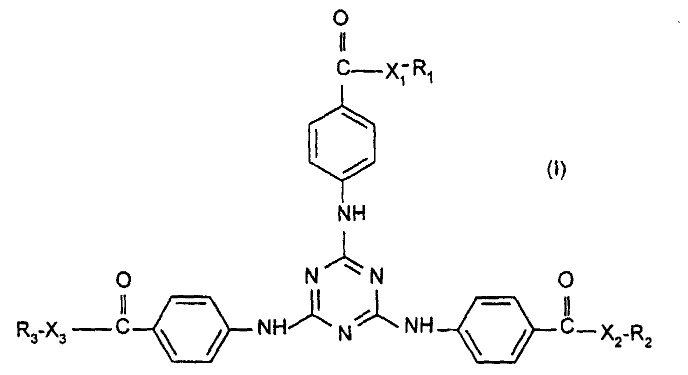 dans laquelle :
- X₁, X₂ et X₃, identiques ou différents, représentent l'oxygène ou un radical -NR-;
- les radicaux R, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- R₁, R₂ et R₃, identiques ou différents, sont choisis parmi : l'hydrogène; un métal alcalin; un radical ammonium éventuellement substitué par un ou plusieurs radicaux alkyle ou hydroxyalkyle; un radical alkyle linéaire ou ramifié en C₁-C₁₈; un radical cycloalkyle en C₅-C₁₂ éventuellement substitué par un ou plusieurs radicaux alkyle en C₁-C₄; un radical polyoxyéthyléné comprenant de 1 à 6 unités d'oxyde d'éthylène et dont le groupe OH terminal est méthylé; un radical de formule (II), (III) ou (IV) suivantes : 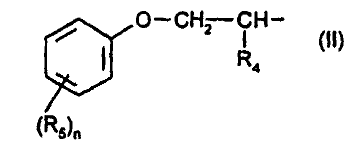 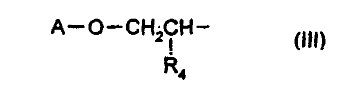 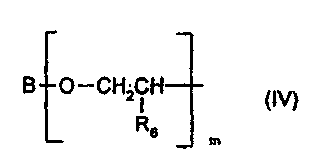
dans lesquelles :
- R₄ est l'hydrogène ou un radical méthyle;
- R₅ est un radical alkyle en C₁-C₉;
- n est un nombre entier allant de 0 à 3;
- m est un nombre entier allant de 1 à 10;
- A est un radical alkyle en C₄-C₈ ou un radical cycloalkyle en C₅-C₈;
- B est choisi parmi : un radical alkyle linéaire ou ramifié en C₁-C₈; un radical cycloalkyle en C₅-C₈; un radical aryle éventuellement substitué par un ou plusieurs radicaux alkyle en C₁-C₄;
- R₆ est l'hydrogéne ou un radical méthyle ;

Une première famille préférée de dérivés de 1,3,5-triazine est celle, notamment décrite dans le document EP-A-0517104 référence dans la présente description) des 1,3,5-triazines répondant à la formule (I) ci-dessus et présentant l'ensemble des caractéristiques suivantes :
- X₁, X₂ et X₃ sont identiques et représentent l'oxygène ;
- R₁ est choisi parmi : un radical cycloalkyle en C₅-C₁₂ éventuellement substitué par un ou plusieurs radicaux alkyles en C₁-C₄; un radical de formule (II), (III) ou (IV) ci-dessus dans lesquelles :
   - B est un radical alkyle en C₁-C₄;
   - R₆ est le radical méthyle;
   - R₂ et R₃, identiques ou différents, sont choisis parmi : l'hydrogène; un métal alcalin; un radical ammonium éventuellement substitué par un ou, plusieurs radicaux alkyle ou hydroxyalkyle; un radical alkyle linéaire ou ramifié en C₁-C₁₈; un radical cycloalkyle en C₅-C₁₂ éventuellement substitué par un ou plusieurs radicaux aikyie en C₁-C₄; un radical de formule (II), (III) ou (IV) ci-dessus dans lesquelles :
      - B est un radical alkyle en C₁-C₄;
      - R₆ est le radical méthyle.

Une deuxième famille préférée de dérivés de 1,3,5-triazine selon l'invention est celle, notamment décrite dans le document EP-A-0570838 des 1,3,5-triazines répondant à la formule (I) et présentant l'ensemble des caractéristiques suivantes :
- X₁ est l'oxygène ; X₂ est le radical NH ou l'oxygène ;
- X₃ est le radical -NH- ;
- R₃ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄;
- R₁ est choisi parmi : l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄;
- si X₂ est le radical -NH-, alors R₂ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄;
- si X₂ est l'oxygène, alors R₂ est choisi parmi l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄.

Une troisième famille préférée de dérivés de 1,3,5-triazine selon l'invention est celle, notamment décrite dans le document EP-A-0796851 des 1,3,5-triazines répondant à la formule (I) et présentant l'ensemble des caractéristiques suivantes :
- X₁, X₂ et X₃ désignent simultanément -NR- ;
- les radicaux R, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- R₁, R₂ et R₃, identiques ou différents, désignent l'hydrogène ou un radical R.

Une 1,3,5-triazine particulièrement préférée selon l'invention est celle répondant à la formule suivante : 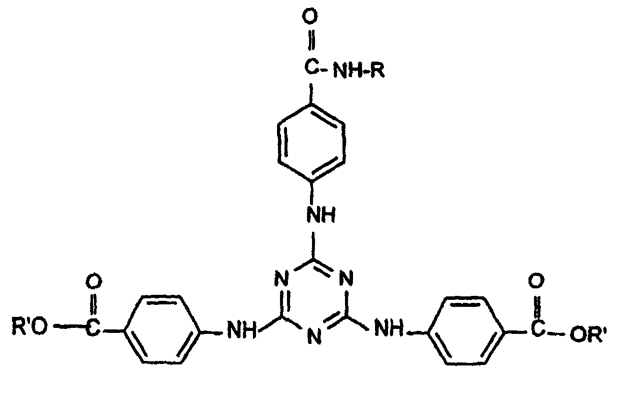 dans laquelle R' désigne un radical éthyl-2 hexyle et R désigne un radical tert-butyle.

Une autre 1,3,5-triazine particulièrement préférée est la 2,4,6-tris[p-(2'-éthylhexyl-1'-oxycarbonyl)anilino]-1,3,5-triazine qui est un filtre connu en soi, actif dans l'UV-B, se présentant sous une forme solide, et qui est vendu notamment sous la dénomination commerciale de "UVINUL T150" par la Société BASF. Ce produit répond à la formule suivante : 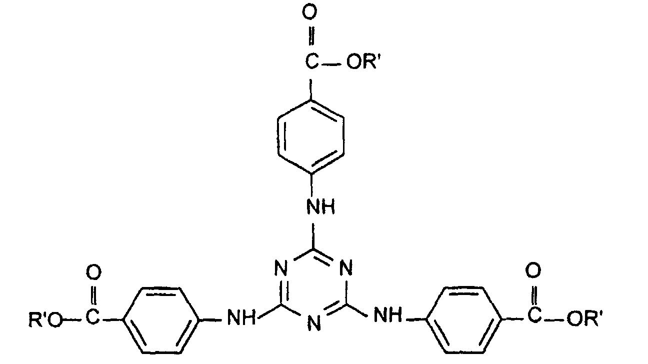 dans laquelle R' désigne un radical 2-éthyl hexyle.

Le ou les dérivés de 1,3,5-triazine sont généralement présents dans les compositions de l'invention à une teneur pouvant aller de 0,5 % à 20 %, de préférence de 1 % à 10 % en poids, par rapport au poids total de la composition.

Les compositions conformes à l'invention comprennent un polyalkyl polyéther siloxane portant des chaînes polyalcoxylées sur la chaîne principale et plus préférentiellement des chaînes polyoxyéthylènes et/ou polyoxypropylènes greffées sur la chaîne principale.

Plus particulièrement, les polyalkyl polyéther siloxanes de l'invention sont choisis parmi les composés de formule générale (V) suivante : 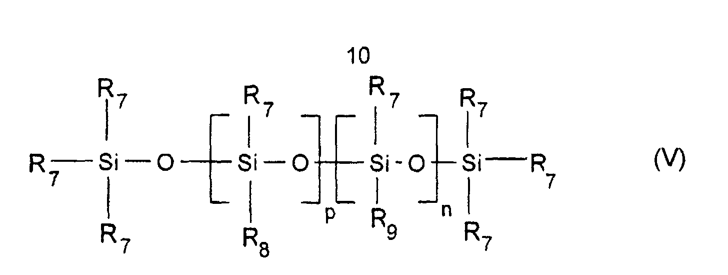 formule dans laquelle :
- R₇ et R₉, identiques ou différents, représentent un atome d'hydrogène, un radical alkyle linéaire ou ramifié en C₁-C₃₀ ou un radical phényle,
- R₈, identiques ou différents, représentent -(CₓH₂ₓ)-(OC₂H₄)ₐ-(OC₃H₆)_{b}-OR₁₀,
- R₁₀, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 1 à 1000,
- p varie de 1 à 30,
- a varie de 1 à 50,
- b varie de 0 à 50,
- x varie de 1 à 5.

Le poids moléculaire moyen en nombre de composé siliconé est en général supérieur ou égal à 15000 et de préférence compris entre 20 000 et 40 000.

Une première famille de polyalkyl polyéther siloxanes convenant particulièrement bien aux compositions selon l'invention est celle des composés répondant à la formule (V) ci-dessus pour lesquels les radicaux R₇ et R₉ sont identiques et représentent tous des radicaux méthyle et le radical R₁₀ représente l'hydrogène.

A titre d'exemple de composé siliconé appartenant à cette famille, on peut citer le poly diméthylsiloxane oxyéthyléné (9/4) (12 OE) fourni par la société Dow Corning sous le nom «DC 193» ; le poly diméthyl/méthyl siloxane oxyéthyléné et oxypropyléné (OE/OP 18/18) pour lequel n est 396 et p est 4, de poids moléculaire en nombre supérieur à 30 000 (nom CTFA : Cyclométhicone 90% Dimethicone copolyol 10%) vendu sous la dénomination commerciale de « Silicone DC 3225C » par la société Dow Corning.

Dans la définition ci-dessus et dans la suite du texte, OE représente une mole d'oxyde d'éthylène et OP représente une mole d'oxyde de propylène.

Une deuxième famille de polyalkyl polyéther siloxanes convenant particulièrement bien aux compositions selon l'invention est celle des composés répondant à la formule (V) ci-dessus pour lesquels les radicaux R₇ représentent tous des radicaux méthyle et les radicaux R₉ représentent tous des radicaux lauryle.

Un composé particulièrement préféré de cette deuxième famille est le poly méthyllauryl/méthyl siloxane oxyéthyléné oxypropyléné (OE/OP 18/18) pour lequel n est 35 et p est 3, de poids moléculaire en nombre supérieur à 25 000 (nom CTFA : Laurylméthicone copolyol 91%, Isostéaryl alcohol 9%) vendu sous la dénomination commerciale «DC Q2-5200» par la société Dow Corning.

Le ou les polyalkyl polyéther siloxanes de l'invention sont généralement présents dans les compositions selon l'invention en une proportion en matière active, comprise entre 0,2% et 5% en poids, de préférence entre 0,25% et 3% en poids, par rapport au poids total de la composition.

Les émutsions huile-dans-eau conformes à l'invention comprennent en général au moins un agent épaississant et/ou gélifiant, hydrosoluble ou hydrophile. Elles contiennent de préférence moins de 0,5% en poids d'agent tensioactif émulsionnant classique par rapport au poids total de la composition et plus particulièrement sont dépourvues d'agent tensioactif émulsionnant classique.

A titre d'agent épaississant et/ou gélifiant utilisable, on peut citer :
- les extraits d'algues tels que l'agar-agar, les carraghénanes, les alginates ;
- les extraits de graines tels que la gomme de caroube, la gomme de guar et leurs dérivés;
- les exsudats de plantes tels que la gomme arabique, la gomme karaya, la gomme adragante, la gomme de gatty ;
- les exsudats de micro-organismes tels que la gomme de xanthane,
- la cellulose ou ses dérivés comme la carboxyméthylcellulose, l'hydroxy-propylcellulose, la méthylcellulose, l'hydroxypropylméthylcellulose ou l'hydroxy-éthylcellulose ainsi que les celluloses modifiées notamment par greffage de groupement alkyle ;
- les extraits de fruits tels que les pectines ;
- les agents gélifiants d'origine animale tels que les caséïnates ;
- les polymères synthétiques ;
- les dérivés du silicium tels que les hectorites synthétiques comme les produits "Laponite RD et RDS" vendus par la société WAVERLY, les silicates d'aluminium et de magnésium comme le produit "Veegum" vendu par la société VANDERBILT.
- les mélanges des composés ci-dessus.

Les polymères synthétiques épaississants et/ou gélifiants préférentiels sont choisis parmi :
(a) les homopolymères d'acide acrylique réticulés par un éther allylique d'alcool de la série du sucre tels que les produits vendus sous les noms CARBOPOLS 980, 981, 954, 2984 et 5984 par la société GOODRICH ou les produits vendus sous les noms SYNTHALEN M et SYNTHALEN K par la société 3 VSA ;
(b) les copolymères réticulés comportant une fraction majoritaire d'acide acrylique et une faible fraction d'esters d'acide (meth)acrylique en C₁₀-C₃₀ tels que les produits vendus sous les noms PEMULEN TR1, PEMULEN TR2 et CARBOPOL 1342 par la société GOODRICH (ils sont décrits et préparés dans le document EP-A-268164) ;
(c) les homopolymères réticulés d'acide 2-acrylamido-2-méthyl-propane sulfonique tels que ceux décrits dans la demande EP-A-0815828 et leurs copolymères réticulés d'acrylamide partiellement ou totalement neutralisés (par une base telle que la soude, de la potasse ou une amine) tels que le produit décrit dans l'exemple 1 du document EP-A-503853 ;
(d) les homopolymères d'acrylate d'ammonium tels que le produit vendu sous le nom MICROSAP PAS 5193 par la société HOECHST ou les copolymères d'acrylate d'ammonium et d'acrylamide tels que le produit vendu sous le nom BOZEPOL C NOUVEAU ou le produit PAS 5193 vendus par la société HOECHST (ils sont décrits et préparés dans les documents FR2416723, USP2798053 et USP 2 923 692) ;
(e) les homopolymères de diméthylaminoéthyl-méthacrylate quaternisé par le chlorure de méthyle tels que les produits vendus sous les noms SALCARE 95 et SALCARE 96 par la société ALLIED COLLOIDS ou les copolymères de diméthylamino-éthylméthacrylate quaternisé par le chlorure de méthyle et d'acrylamide tel que le produit SALCARE SC92 vendu par ALLIED COLLOIDS ou le produit PAS 5194 vendu par HOECHST (ils sont décrits et préparés dans le document EP-A-395282).

Les agents épaississants et/ou gélifiants sont utilisés dans des concentrations allant de préférence de 0,1 à 10% en poids, en particulier de 0,1 à 5% en poids par rapport au poids total de la composition.

Les compositions selon l'invention comprennent également une phase huileuse qui peut comporter un ou plusieurs corps gras, ces corps gras pouvant être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline), les huiles végétales (huile d'amande douce, de macadamia, de pépin de cassis, de jojoba), les huiles synthétiques comme le perhydrosqualène, les alcools, les acides ou les esters gras (comme le benzoate d'alcools en C₁₂-C₁₅ vendu sous la dénomination commerciale «Finsolv TN» par la société Finetex, le palmitate d'octyle, le lanolate d'isopropyle, les triglycérides dont ceux des acides caprique/caprylique), les esters et éthers gras oxyéthylénés ou oxypropylénés, les huiles fluorées ou encore les polyalkylènes comme le polydécène.

Comme composés cireux, on peut citer la paraffine, la cire de carnauba, la cire d'abeille, l'huile de ricin hydrogénée.

La phase huileuse peut également comprendre une huile de silicone, volatile ou non telle que les cyclométhicones ou les diméthicones. On peut par exemple mettre en oeuvre dans les compositions de la présente invention une huile de silicone volatile comme par exemple les cyclométhicones vendues sous les dénominations commerciales «DC245 Fluid» ou «DC 246 Fluid» par Dow Corning.

Les compositions cosmétiques et/ou dermatologiques visées par la présente invention peuvent bien entendu contenir un ou plusieurs filtres solaires actifs dans l'UVA et/ou l'UVB (absorbeurs), hydrophiles ou lipophiles. Ces filtres peuvent être notamment choisis parmi les dérivés cinnamiques, les dérivés salicyliques, les dérivés du dibenzoylméthane, les dérivés du benzylidène camphre, les dérivés de benzimidazole, les dérivés de la benzophénone, les dérivés de β,β'-diphénylacrylate, les dérivés de l'acide p-amino-benzoïque, les polymères filtres et silicones filtres décrits dans la demande WO-93/04665. D'autres exemples de filtres organiques sont donnés dans la demande de brevet EP-A0487404.

Comme filtres hydrophiles particulièrement utilisables dans la présente invention, on peut citer l'acide benzène 1,4-di(3-méthylidène-10-camphosulfonique) et l'acide 2-phényl-benzimidazole-5-sulfonique vendu sous la dénomination commerciale «Eusolex 232» par la société Merck ou leur mélange.

Comme filtres lipophiles particulièrement utilisables dans la présente invention, on peut citer le 4-tert-butyl-4'-méthoxydibenzoylméthane vendu sous la dénomination commerciale «Parsol 1789» par la société Givaudan et/ou l'α-cyano-β, β-diphényl-acrylate de 2-éthylhexyle vendu sous la dénomination commerciale «Uvinul N 539» par la société BASF.

Le ou les filtres hydrophiles peuvent être présents dans la composition finale selon l'invention à une teneur pouvant varier de 0,1 à 20%, de préférence de 0,2 à 10%, en poids, par rapport au poids total de la composition. Le ou les filtres lipophiles peuvent être présents dans la composition finale selon l'invention à une teneur pouvant varier de 0,5 à 30%, de préférence de 0,5 à 20%, en poids, par rapport au poids total de la composition.

Les compositions cosmétiques et/ou dermatologiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium, les silicones. De tels nanopigments d'oxydés métalliques, enrobés ou non enrobés, sont en particulier décrits dans les demande de brevets EP-A-0518772 et EP-A- 0518773.

Les nanopigments peuvent être présents dans la composition finale selon l'invention à une teneur pouvant varier de 0,1 à 20%, de préférence de 0,2 à 10%, en poids, par rapport au poids total de la composition.

Les compositions selon l'invention peuvent également contenir des agents de bronzage et/ou de brunissage artificiels de la peau (agents autobronzants), tels que par exemple de la dihydroxyacétone (DHA).

Les compositions conformes à la présente invention peuvent comprendre en outre des adjuvants cosmétiques et/ou dermatologiques classiques notamment choisis parmi les solvants organiques, les adoucissants, les antioxydants, les agents anti radicaux libres, les opacifiants, les stabilisants, les émollients, les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Bien entendu, l'homme de l'art veillera à choisir le ou les éventuels composés complémentaires cités ci-dessus et/ou leurs quantités de manière telle que les propriétés avantageuses attachées intrinsèquement à l'association binaire conforme à l'invention ne soient pas, ou substantiellement pas, altérées par la ou les adjonctions envisagées.

Les compositions selon l'invention peuvent être préparées selon les techniques de préparation d'émulsions de type huile-dans-eau qui sont bien connues de l'homme de l'art.

La composition cosmétique et/ou dermatologique de l'invention peut être utilisée comme composition protectrice de l'épiderme humain et/ou des cheveux et/ou les autres matières kératiniques humaines telles que les cils, les sourcils ou les ongle contre les rayons ultraviolets, comme composition antisolaire ou comme produit de maquillage.

Des exemples concrets, mais nullement limitatifs, illustrant l'invention, vont maintenant être donnés.

### EXEMPLE 1 : Emulsion huile-dans-eau

- 2,4,6-tris[p-(2'-éthylhexyl-1'-oxycarbonyl)anilino]-1,3,5triazine vendu sous le nom « UVINUL T 150 » par la Société BASF 2,5% en poids
- Poly diméthylsiloxane oxyéthyléné (9/4) (12 OE) fourni par la société Dow Corning sous le nom «DC 193» 1,5% en poids
- Copolymère réticulé d'acide 2-acrylamido-2-méthy-propane sulfonique/acrylamide vendu sous le nom "SEPIGEL 305" par la Société SEPPIC 4,0% en poids
- Benzoate d'alcools en C₁₂/C₁₅ vendu sous la dénomination commerciale « Finsolv TN » par Finetex 25 % en poids
- Hydratants 12 % en poids
- Alcool dénaturé 4,5% en poids
- Conservateurs qs
- Eau purifiée qsp 100% en poids

La formulation ne se déphase pas après centrifugation à 3000 tours/minute pendant 30 minutes et reste stable après deux mois de stockage à 45°C.

### EXEMPLE 2 (ne faisant pas partie de l'invention) : Emulsion huile-dans-eau

- Poly diméthylsiloxane oxyéthyléné (9/4) (12 OE) fourni par la société Dow Corning sous le nom «DC 193» 1,5% en poids
- Copolymère réticulé d'acide 2-acrylamido-2-méthyl-propane sulfoniquel acrylamide vendu sous le nom «SEPIGEL 305» par la Société SEPPIC 4,0% en poids
- Benzoate d'alcools en C₁₂/C₁₅ vendu sous la dénomination commerciale «Finsolv TN» par Finetex 27,5% en poids
- Hydratants 12 % en poids
- Alcool dénaturé 4,5% en poids
- Conservateurs qs
- Eau purifiée qsp 100% en poids

La formulation relargue après centrifugation à 3000 tours/minute pendant 30 minutes et après deux mois de stockage à 45°C, on observe une séparation de phases.

### EXEMPLE 3 : Composition solaire

- 2,4,6-tris[p-(2'-éthylhexyl-1'-oxycarbonyl)anilino]-1,3,5-triazine vendu sous le nom «UVINUL T 150» par la Société BASF 2,0% en poids
- α-cyano-β, β-diphénylacrylate de 2-éthylhexyle vendu sous la dénomination commerciale «Uvinul N 539» par BASF 10 % en poids
- Octyl méthoxycinnamate vendu sous la dénomination commerciale «PARSOL MCX» par la société GIVAUDAN 5,0 % en poids
- Acide benzène 1,4-di(3-méthylidène-10-camphosulfonique) 0,5 % en poids
- Isononanoate d'isononyle 2,0% en poids
- Silicone filtre benzotriazole de formule suivante : 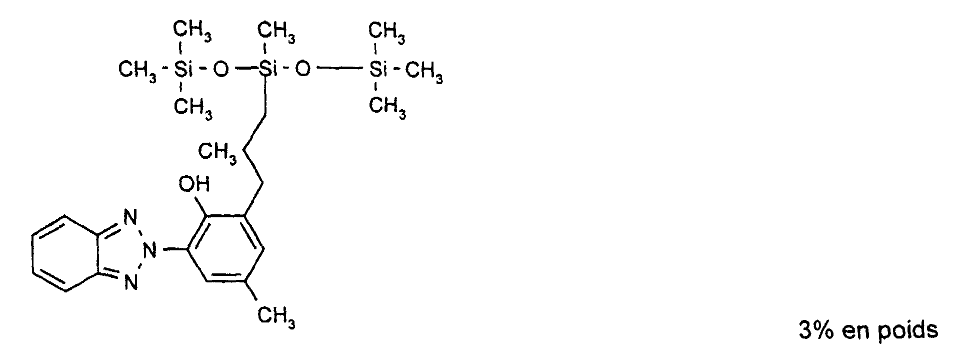
- Mélange polydiméthylsiloxane α,ω-dihydroxyle/polydiméthyl siloxane vendu sous le nom « DOW CORNING DC 1403 FLUID » par la société Dow Corning 4,0% en poids
- Poly diméthylsiloxane oxyéthyléné (9/4) (12 OE) fourni par la société Dow Corning sous le nom «DC 193» 1,5% en poids
- Cocoglycérides vendus sous le nom «MYRITOL 331» par la société HENKEL 3,0% en poids
- Copolymère réticulé d'acide 2-acrylamido-2-méthyl-propane sulfonique/acrylamide vendu sous le nom «SEPIGEL 305» par la Société SEPPIC 4,0% en poids
- Hydratants 12 % en poids
- Conservateurs qs
- Triéthanolamine qs pH 7
- Eau purifiée qsp 100% en poids

La formulation ne relargue pas après centrifugation à 3000 tours/minute pendant 30 minutes et reste stable après deux mois de stockage à 45°C.

### EXEMPLE 4 (ne faisant pas partie de l'invention): Composition solaire

- α-cyano-β, β-diphénylacrylate de 2-éthylhexyle vendu sous la dénomination commerciale « Uvinul N 539 » par BASF 10 % en poids
- Octyl méthoxycinnamate vendu sous la dénomination commerciale « PARSOL MCX » par la société GIVAUDAN 7,0 % en poids
- Acide benzène 1,4-di(3-méthylidène-10-camphosulfonique) 0,5 % en poids
- Isononanoate d'isononyle 2,0% en poids
- Silicone filtre benzotriazole de formule suivante : 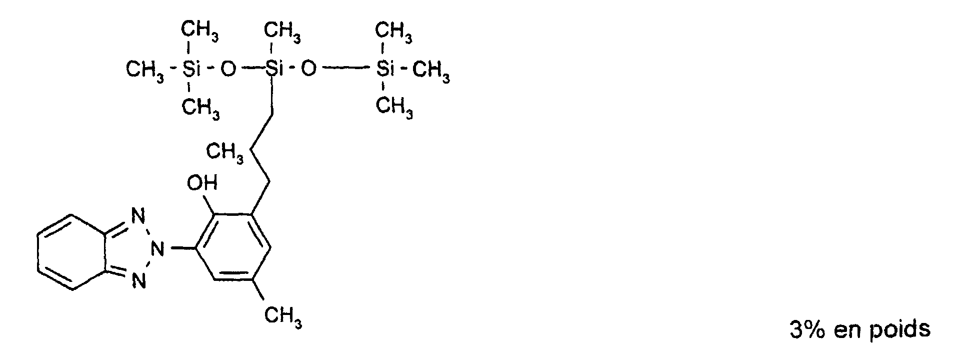
- Mélange polydiméthylsiloxane α,ω-dihydroxyle/polydiméthylsiloxane vendu sous le nom « DOW CORNING DC 1403 FLUID » par la société Dow Corning 4,0% en poids
- Poly diméthylsiloxane oxyéthyléné (9/4) (12 OE) fourni par la société Dow Corning sous le nom « DC 193 » 1,5% en poids
- Cocoglycérides vendus sous le nom « MYRITOL 331 » par la société HENKEL 3,0% en poids
- Copolymère réticulé d'acide 2-acrylamido-2-méthyl - propane sulfonique/acrylamide vendu sous le nom « SEPIGEL 305 » par la Société SEPPIC 4,0% en poids
- Hydratants 12 % en poids
- Conservateurs qs
- Triéthanolamine qs pH 7
- Eau purifiée qsp 100% en poids

La formulation relargue après deux mois de stockage à 45°C. Le remplacement dans l'exemple 4 du dérivé de triazine (2% en poids) par de l'octyl méthoxycinnamate ne permet pas de stabiliser l'émulsion huile-dans-eau contenant la silicone copolyol.

### EXEMPLE 5 : Composition solaire

- 2,4,6-tris[p-(2'-éthylhexyl-1'-oxycarbonyl)anilino]-1,3,5-triazine vendu sous le nom « UVINUL T 150 » par la Société BASF 1,0% en poids
- α-cyano-β, β-diphénylacrylate de 2-éthylhexyle vendu sous la dénomination commerciale «Uvinul N 539» par BASF 2,1 % en poids
- 4-tert-butyl-4'-méthoxydibenzoylméthane vendu sous la dénomination commerciale « PARSOL 1789 » par GIVAUDAN 0,9 % en poids
- Acide benzène 1,4-di(3-méthylidène-10-camphosulfonique) 0,5 % en poids
- Heptanoate de stéaryle 2,0% en poids
- Dioxyde de titane (UV TITAN M160 de KENMIRA) 0,5% en poids
- Copolymère acide acrylique / acrylate d'alkyles en C₁₀-C₃₀ vendu sous la dénomination commerciale «PEMULEN TR-1 » par la société GOODRICH 0,4 % en poids
- Hexa décyl phosphate de K vendu sous le nom commercial «AMPHISOL K» par ROCHE 0,2 % en poids
- Poly diméthyl / méthyl siloxane oxyéthyléné oxypropyléné (396/4) (OE/OP 18/18) fourni sous le nom «DC 3225 C» par la société Dow Corning 1,0% en poids
- Silicone volatile 5,0% en poids
- Stéarate oligomère d'acide 12-hydroxy stéarique vendu sous le nom SOLSPERSE 21000 0,05 % en poids
- Benzoate d'alcools en C₁₂/C₁₅ vendu sous la dénomination commerciale «Finsolv TN» par Finetex 6,0 % en poids
- Hydratants 12 % en poids
- Conservateurs qs
- Triéthanolamine qs pH 7
- Eau purifiée qsp 100% en poids

La formulation ne relargue pas après centrifugation à 3000 tours/minute pendant 30 minutes et reste stable après deux mois de stockage à 45°C.

### EXEMPLE 6 (ne faisant pas partie de l'invention : Composition solaire

- α-cyano-β, β-diphénylacrylate de 2-éthylhexyle vendu sous la dénomination commerciale «Uvinul N 539» par BASF 2,1 % en poids
- 4-tert-butyl-4'-méthoxydibenzoylméthane vendu sous la dénomination commerciale «PARSOL 1789» par GIVAUDAN 0,9 % en poids
- Acide benzène 1,4-di(3-méthylidène-10-camphosulfonique) 0,5 % en poids
- Heptanoate de stéaryle 2,0% en poids
- Dioxyde de titane (UV TITAN M160 de KENMIRA) 0,5% en poids
- Copolymère acide acrylique / acrylate d'alkyles en C₁₀-C₃₀ vendu sous la dénomination commerciale«PEMULEN TR-1» par la société GOODRICH 0,4 % en poids
- Hexa décyl phosphate de K vendu sous le nom commercial «AMPHISOL K» par ROCHE 0,2 % en poids
- Poly diméthyl / méthyl siloxane oxyéthyléné oxypropyléné (396/4) (OE/OP 18/18) fourni sous le nom «DC 3225 C» par la société Dow Corning 1,0% en poids
- Silicone volatile 5,0% en poids
- Stéarate oligomère d'acide 12-hydroxy stéarique vendu sous le nom SOLSPERSE 21000 0,05 % en poids
- Benzoate d'alcools en C₁₂/C₁₅ vendu sous la dénomination commerciale « Finsolv TN » par Finetex 7,0 % en poids
- Hydratants 12 % en poids
- Conservateurs qs
- Triéthanolamine qs pH 7
- Eau purifiée qsp 100% en poids

La formulation relargue après centrifugation à 3000 tours/minute pendant 30 minutes.

## Revendications

1. Emulsion huile-dans-eau, **caractérisée par le fait qu'**elle comprend au moins :
i) un dérivé de 1,3,5-triazine de formule suivante : 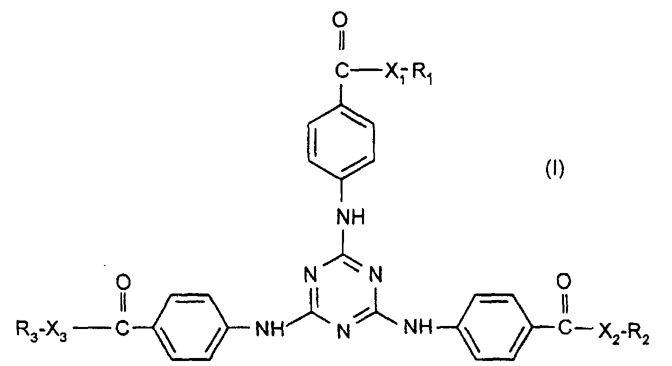 dans laquelle :
- X₁, X₂ et X₃, identiques ou différents, représentent l'oxygène ou un radical-NR-;
- les radicaux R, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- R₁, R₂ et R₃, identiques ou différents, sont choisis parmi : l'hydrogène; un métal alcalin; un radical ammonium éventuellement substitué par un ou plusieurs radicaux alkyle ou hydroxyalkyle; un radical alkyle linéaire ou ramifié en C₁-C₁₈; un radical cycloalkyle en C₅-C₁₂ éventuellement substitué par un ou plusieurs radicaux alkyle en C₁-C₄; un radical polyoxyéthyléné comprenant de 1 à 6 unités d'oxyde d'éthylène et dont le groupe OH terminal est méthylé; un radical de formule (II), (III) ou (IV) suivantes : 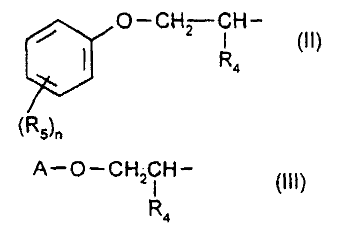 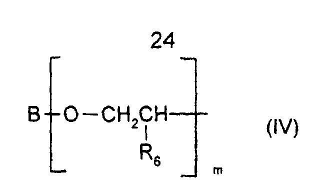
dans lesquelles :
- R₄ est l'hydrogène ou un radical méthyle ;
- R₅ est un radical alkyle en C₁-C₉ ;
- n est un nombre entier allant de 0 à 3 ;
- m est un nombre entier allant de 1 à 10 ;
- A est un radical alkyle en C₄-C₈ ou un radical cycloalkyle en C₅-C₈;
- B est choisi parmi : un radical alkyle linéaire ou ramifié en C₁-C₈; un radical cycloalkyle en C₅-C₈; un radical aryle éventuellement substitué par un ou plusieurs radicaux alkyle en C₁-C₄;
- R₆ est l'hydrogène ou un radical méthyle ;
ii) un polyalkyl polyéther siloxane portant des groupes polyoxyalkylènés greffés sur la chaîne siliconée principale; sous réserve que la dite émulsion ne contient pas de chlorure de cétylstéaryltriméthylammonium.

2. Emulsion selon la revendication 1, où les dérivés de 1,3,5-triazine de formule (I) présentent l'ensemble des caractéristiques suivantes :
- X₁, X₂ et X₃ sont identiques et représentent l'oxygène ;
- R₁ est choisi parmi : un radical cycloalkyle en C₅-C₁₂ éventuellement substitué par un ou plusieurs radicaux alkyles en C₁-C₄; un radical de formule (II), (III) ou (IV) ci-dessus dans lesquelles :
- B est un radical alkyle en C₁-C₄;
- R₆ est le radical méthyle;
- R₂ et R₃, identiques ou différents, sont choisis parmi : l'hydrogène; un métal alcalin; un radical ammonium éventuellement substitué par un ou plusieurs radicaux alkyle ou hydroxyalkyle; un radical alkyle linéaire ou ramifié en C₁-C₁₈; un radical cycloalkyle en C₅-C₁₂ éventuellement substitué par un ou plusieurs radicaux alkyle en C₁-C₄; un radical de formule (II), (III) ou (IV) ci-dessus dans lesquelles :
- B est un radical alkyle en C₁-C₄;
- R₆ est le radical méthyle.

3. Emulsion selon la revendication 1, où les dérivés de 1,3,5-triazine de formule (I) présentent l'ensemble des caractéristiques suivantes :
- X₁ est l'oxygène ; X₂ est le radical NH ou l'oxygène ;
- X₃ est le radical -NH- ;
- R₃ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- R₁ est choisi parmi : l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄ ;
- si X₂ est le radical -NH-, alors R₂ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- si X₂ est l'oxygène, alors R₂ est choisi parmi l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄.

4. Emulsion selon la revendication 1, où les dérivés de 1,3,5-triazine de formule (I) présentent l'ensemble des caractéristiques suivantes :
- X₁, X₂ et X₃ désignent simultanément -NR- ;
- les radicaux R, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- R₁, R₂ et R₃, identiques ou différents, désignent l'hydrogène ou un radical R.

5. Emulsion selon la revendication 1, où le dérivé de 1,3,5-triazine répond à la formule suivante : 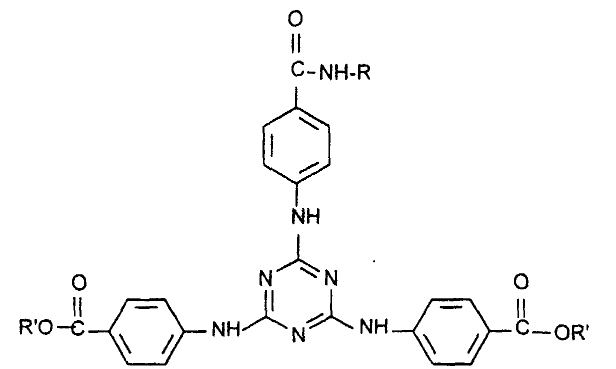 dans laquelle R' désigne un radical éthyl-2 hexyle et R désigne un radical tert-butyle.

6. Emulsion selon la revendication 1, où le dérivé de 1,3,5-triazine répond à la formule suivante : 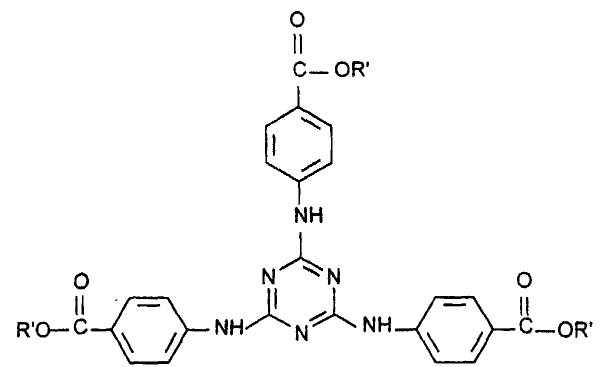 dans laquelle R' désigne un radical 2-éthyl hexyle.

7. Emulsion selon l'une quelconque des revendications 1 à 6, où les dérivés Le de 1,3,5-triazine sont présents à une teneur allant de 0,5% à 20%, de préférence de 1% à 10% en poids, par rapport au poids total de la composition.

8. Emulsion selon l'une quelconque des revendications 1 à 7, où les polyalkyl polyéther siloxanes comportent des chaînes polyoxyéthylènes et/ou polyoxypropylènes greffées sur la chaîne principale.

9. Emulsion selon l'une quelconque des revendications 1 à 7, où les polyalkyl polyéther siloxanes sont choisis parmi les composés de formule générale (V) suivante : 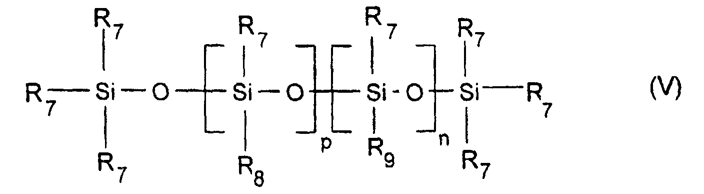 formule dans laquelle :
- R₇ et R₉, identiques ou différents, représentent un atome d'hydrogène, un radical alkyle linéaire ou ramifié en C₁-C₃₀ ou un radical phényle,
- R₈, identiques ou différents, représentent -(CₓH₂ₓ)-(OC₂H₄)ₐ-(OC₃H₆)_{b}-OR₁₀,
- R₁₀, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 1 à 1000,
- p varie de 1 à 30,
- a varie de 1 à 50,
- b varie de 0 à 50,
- x varie de 1 à 5.

10. Emulsion selon l'une quelconque des revendications 1 à 7, où les polyalkyl polyéther siloxanes présentent un poids moléculaire moyen en nombre supérieur ou égal à 15000 et de préférence allant de 20 000 à 40 000.

11. Emulsion selon l'une quelconque des revendications 9 à 10, où les polyalkyl polyéther siloxanes répondent à la formule (V) dans laquelle les radicaux R₇ et R₉ sont identiques et représentent tous des radicaux méthyle et le radical R₁₀ représente l'hydrogène.

12. Emulsion selon l'une quelconque des revendications 9 à 10, où les polyalkyl polyéther siloxanes répondent à la formule (V) dans laquelle les radicaux R₇ représentent tous des radicaux méthyle et les radicaux R₉ représentent tous des radicaux lauryle.

13. Emulsion selon l'une quelconque des revendications 1 à 12, où les polyalkyl polyéther siloxanes sont présents dans une proportion en matière active, allant de 0,2% à 5% en poids, de préférence de 0,25% et 3% en poids, par rapport au poids total de la composition.

14. Emulsion selon l'une quelconque des revendications 1 à 13, contenant en plus au moins un agent épaississant et/ou gélifiant, hydrosoluble ou hydrophile.

15. Emulsion selon l'une quelconque des revendications 1 à 14, **caractérisée par le fait qu'**elle contient moins de 0,5 % en poids d'agent tensioactif émulsionnant classique.

16. Emulsion selon l'une quelconque des revendications 1 à 15, **caractérisée par le fait qu'**elle ne contient pas d'agent tensioactif émulsionnant classique.

17. Emulsion selon la revendication 14, où l'agent épaississant et/ou gélifiant est choisi parmi :
- les extraits d'algues ;
- les extraits de graines ;
- les exsudats de plantes ;
- les exsudats de micro-organismes ;
- la cellulose ou ses dérivés ainsi que les celluloses modifiées ;
- les extraits de fruits ;
- les agents gélifiants d'origine animale ;
- les polymères synthétiques ;
- les dérivés du silicium ;
- les mélanges des composés ci-dessus.

18. Emulsion selon la revendication 17, où les polymères synthétiques épaississants et/ou gélifiant sont choisis parmi :
(a) les homopolymères d'acide acrylique réticulés par un éther allylique d'alcool de la série du sucre ;
(b) les copolymères réticulés comportant une fraction majoritaire d'acide acrylique et une faible fraction d'esters d'acide (meth)acrylique en C₁₀-C₃₀ ;
(c) les homopolymères réticulés d'acide 2-acrylamido-2-méthyl-propane sulfonique et leurs copolymères réticulés d'acrylamide partiellement ou totalement neutralisés ;
(d) les homopolymères d'acrylate d'ammonium ou les copolymères d'acrylate d'ammonium et d'acrylamide ;
(e) les homopolymères de diméthylaminoéthyl-méthacrylate quaternisé par le chlorure de méthyle ou les copolymères de diméthylamino-éthylméthacrylate quaternisé par le chlorure de méthyle et d'acrylamide.

19. Emulsion selon l'une quelconque des revendications 14 à 18, où les agents épaississants et/ou gélifiants sont utilisés dans des concentrations allant de 0,1 à 10 % en poids, en particulier de 0,1 à 5 % en poids par rapport au poids total de la composition.

20. Utilisation d'une émulsion huile-dans-eau selon l'une quelconque des revendications 1 à 19 comme, ou pour la fabrication de, compositions cosmétiques ou dermatologiques destinées à la protection de la peau et/ou des cheveux et les autres matières kératiniques humaines contre le rayonnement ultraviolet, en particulier le rayonnement solaire.

21. Composition cosmétique et/ou dermatologique, **caractérisée par le fait qu'**elle contient au moins une émulsion huile-dans-eau selon l'une quelconque des revendications 1 à 19.

22. Composition selon la revendication 21, contenant en plus un ou plusieurs un ou plusieurs filtres solaires actifs dans l'UVA et/ou l'UVB, hydrophiles ou lipophiles.

23. Composition selon la revendication 22, où les filtres additionnels sont choisis parmi les dérivés cinnamiques, les dérivés salicyliques, les dérivés du dibenzoylméthane, les dérivés du benzylidène camphre, les dérivés de benzimidazole, les dérivés de la benzophénone, les dérivés de β,β'-diphényl-acrylate, les dérivés de l'acide p-aminobenzoïque, les polymères filtres et silicones filtres.

24. Composition selon la revendication 22 ou 23, où le filtre hydrophile additionnel est l'acide benzène 1,4-di(3-méthylidène-10-camphosulfonique) et/ou l'acide 2-phénylbenzimidazole-5-sulfonique.

25. Composition selon la revendication 22 ou 23, où le filtre lipophile additionnel est le 4-tert-butyl-4'-méthoxydibenzoylméthane et/ou l'α-cyano-β,β-diphénylacrylate de 2-éthylhexyle.

26. Composition selon l'une quelconque des revendications 21 à 24, contenant en plus au moins un pigment ou nanopigment d'oxyde métallique enrobé ou non.

27. Composition selon l'une quelconque des revendications 21 à 24, contenant en plus au moins un agent de bronzage et/ou de brunissage artificiel de la peau.

28. Procédé de traitement cosmétique pour la protection de la peau et/ou des cheveux et les autres matières kératiniques humaines contre le rayonnement ultraviolet, en particulier le rayonnement solaire, et qui consiste essentiellement à appliquer sur ces derniers une quantité efficace d'une émulsion huile-dans-eau telle que définie selon l'une quelconque des revendications 1 à 20 ou d'une composition telle que définie selon l'une quelconque des revendications 21 à 27.

29. Utilisation des dérivés de 1,3,5-triazine tels que définis selon l'une quelconque des revendications 1 à 6 comme agent stabilisant pour la préparation d'une émulsion huile-dans-eau contenant au moins un polyalkyl polyéther siloxane portant des groupes polyoxyalkylénés greffés sur la chaîne siliconée tels que définis selon l'une quelconque des revendications 1,8 à 12.

## Patentansprüche

1. Öl-in-Wasser-Emulsion, **dadurch gekennzeichnet, dass** sie zumindest enthält:
i) ein 1,3,5-Triazinderivat der folgenden Formel: 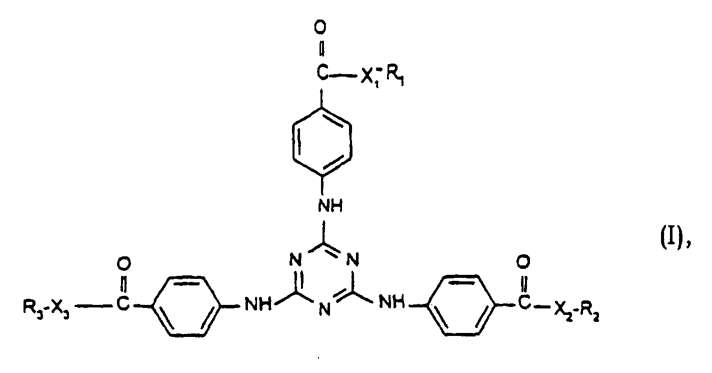 worin:
- die Gruppen X₁, X₂ und X₃, die identisch oder voneinander verschieden sind, Sauerstoff oder eine Gruppe -NR- bedeuten;
- die Gruppen R, die identisch oder voneinander verschieden sind, Wasserstoff, eine geradkettige oder verzweigte C₁₋₁₈-Alkylgruppe oder eine C₅₋₁₂-Cycloalkylgruppe bedeuten, die mit einer oder mehreren C₁₋₄-Alkylgruppen substituiert sein kann;
- die Gruppen R₁, R₂ und R₃, die identisch oder voneinander verschieden sind, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer polyethoxylierten Gruppe, die 1 bis 6 Ethylenoxideinheiten aufweist und deren OH-Endgruppe methyliert ist; einer Gruppe der folgenden Formeln (II), (III) oder (IV): 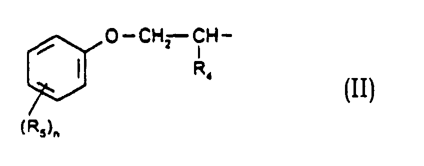 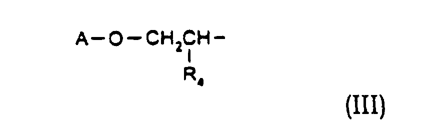 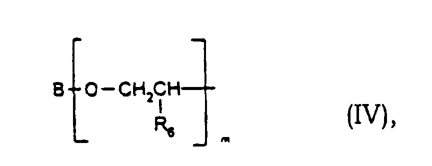
worin bedeuten:
- R₄ Wasserstoff oder Methyl;
- R₅ eine C₁₋₉-Alkylgruppe;
- n Null oder eine ganze Zahl im Bereich von 1 bis 3;
- m eine ganze Zahl im Bereich von 1 bis 10;
- A eine C₄₋₈-Alkylgruppe oder eine C₅₋₈-Cycloalkylgruppe;
- B eine geradkettige oder verzweigte C₁₋₈-Alkylgruppe; eine C₅₋₈-Cycloalkylgruppe; eine Arylgruppe, die gegebenenfalls mit einer oder mehreren C₁₋₄-Alkylgruppen substituiert ist;
- R₆ Wasserstoff oder Methyl; und
ii) mindestens ein Polyalkylpolyethersiloxan, das auf die Siliconhauptkette gepfropfte polyalkoxylierte Gruppen aufweist,
mit der Maßgabe, dass die Emulsion kein Cetylstearyltrimethylammoniumchlorid enthält.

2. Emulsion nach Anspruch 1, wobei die 1,3,5-Triazinderivate der Formel (I) die gesamten folgenden Eigenschaften aufweisen:
- X₁, X₂ und X₃ sind identisch und bedeuten Sauerstoff;
- R₁ ist ausgewählt unter: einer C₅₋₁₂-Cycloalkylgruppe, die gegebenenfalls mit einer oder mehreren C₁₋₄-Alkylgruppen substituiert ist; oder einer oben definierten Gruppe der Formel (II), (III) oder (IV), worin bedeuten:
- B eine C₁₋₄-Alkylgruppe; und
- R₆ die Methylgruppe;
- die Gruppen R₂ und R₃, die identisch oder verschieden sind, sind ausgewählt unter: Wasserstoff; einem Alkalimetall; einer Ammoniumgruppe, die gegebenenfalls mit einer oder mehreren Alkyloder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der oben definierten Formel (II), (III) oder (IV), worin bedeuten:
- B eine C₁₋₄-Alkylgruppe; und
- R₆ die Methylgruppe.

3. Emulsion nach Anspruch 1, wobei die 1,3,5-Triazinderivate der Formel (I) die gesamten folgenden Eigenschaften aufweisen:
- X₁ bedeutet Sauerstoff; X₂ bedeutet -NH- oder Sauerstoff;
- X₃ bedeutet die Gruppe -NH-;
- R₃ ist ausgewählt unter: einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist;
- R₁ ist ausgewählt unter: Wasserstoff; einem Alkalimetall; einer Ammoniumgruppe; einer Gruppe der Formel (IV); einer geradkettigen oder verzweigten C₁₋₁₈-Alkylgruppe; oder einer C₅₋₁₂-Cycloalkylgruppe, die gegebenenfalls mit einer oder mehreren C₁₋₄-Alkylgruppen substituiert ist;
- wenn X₂ die Gruppe -NH- bedeutet, dann ist R₂ ausgewählt unter einer geradkettigen oder verzweigten C₁₋₁₈-Alkylgruppe; oder einer C₅₋₁₂-Cycloalkylgruppe, die gegebenenfalls mit einer oder mehreren C₁₋₄-Alkylgruppen substituiert ist;
- wenn X₂ Sauerstoff bedeutet, dann ist R₂ ausgewählt unter Wasserstoff; einem Alkalimetall; einer Ammoniumgruppe; einer Gruppe der Formel (IV); einer geradkettigen oder verzweigten C₁₋ ₁₈-Alkylgruppe; oder einer C₅₋₁₂-Cycloalkylgruppe, die gegebenenfalls mit einer oder mehreren C₁₋₄-Alkylgruppen substituiert ist.

4. Emulsion nach Anspruch 1, wobei die 1,3,5-Triazinderivate der Formel (I) die gesamten folgenden Eigenschaften aufweisen:
- X₁, X₂ und X₃ sind identisch und bedeuten -NR-;
- die Gruppen R, die identisch oder voneinander verschieden sind, bedeuten Wasserstoff, eine geradkettige oder verzweigte C₁₋₁₈-Alkylgruppe; oder eine C₅₋₁₂-Cycloalkylgruppe, die gegebenenfalls mit einer oder mehreren C₁₋₄-Alkylgruppen substituiert sein kann.
- die Gruppen R₁, R₂ und R₃, die identisch oder voneinander verschieden sind, bedeuten Wasserstoff oder eine Gruppe R.

5. Emulsion nach Anspruch 1, wobei das 1,3,5-Triazinderivat der folgenden Formel entspricht: 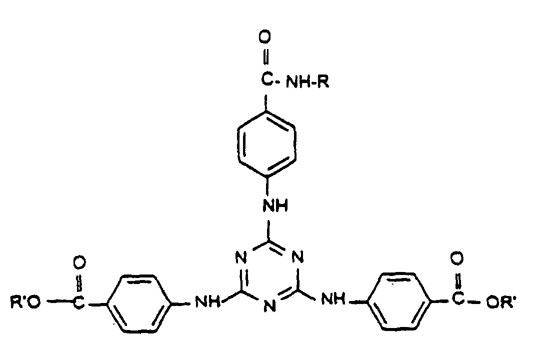 worin die Gruppen R' die 2-Ethylhexylgruppe bedeuten und die Gruppe R *t*-Butyl ist.

6. Emulsion nach Anspruch 1, wobei das 1,3,5-Triazinderivat der folgenden Formel entspricht: 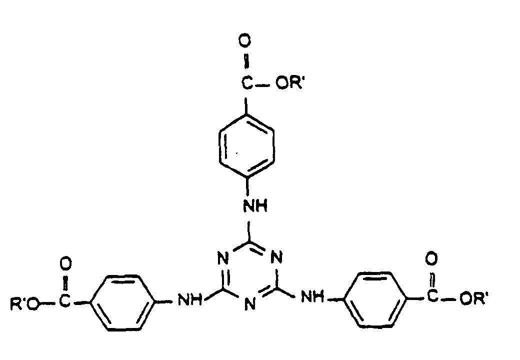 worin die Gruppen R' die 2-Ethylhexylgruppe bedeuten.

7. Emulsion nach einem der Ansprüche 1 bis 6, wobei die 1,3,5-Triazinderivate in einer Menge von 0,5 bis 20 Gew.-% und vorzugsweise 1 bis 10 Gew.-%, bezogen auf das Gesamtgewicht der Zusammensetzung, vorliegen.

8. Emulsion nach einem der Ansprüche 1 bis 7, wobei die Polyalkylpolyethersiloxane auf die Hauptkette gepfropfte Polyethylenoxidund/oder Polypropylenoxidgruppen enthalten.

9. Emulsion nach einem der Ansprüche 1 bis 7, wobei die Polyalkylpolyethersiloxane unter den Verbindungen der allgemeinen Formel (V) ausgewählt sind: 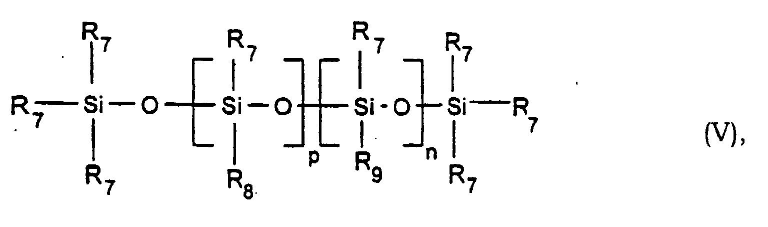 worin:
- die Gruppen R₇ und R₉, die identisch oder voneinander verschieden sind, ein Wasserstoffatom, eine geradkettige oder verzweigte C₁₋₃₀-Alkylgruppe oder eine Phenylgruppe bedeuten,
- die Gruppen R₈, die identisch oder voneinander verschieden sind, - (CₓH₂ₓ)-(OC₂H₄)ₐ-(OC₃H₆)_{b}-OR₁₀ bedeuten,
- die Gruppen R₁₀, die identisch oder voneinander verschieden sind, unter Wasserstoff, einer geradkettigen oder verzweigten Alkylgruppe mit 1 bis 12 Kohlenstoffatomen oder einer geradkettigen oder verzweigten Acylgruppe mit 2 bis 12 Kohlenstoffatomen ausgewählt sind,
- n im Bereich von 1 bis 1000 liegt,
- p im Bereich von 1 bis 30 liegt,
- a im Bereich von 1 bis 50 liegt,
- b im Bereich von 0 bis 50 liegt, und
- x im Bereich von 1 bis 5 liegt.

10. Emulsion nach einem der Ansprüche 1 bis 7, wobei die Polyalkylpolyethersiloxane ein Zahlenmittel der Molmasse von mindestens 15 000 und liegt vorzugsweise im Bereich von 20 000 bis 40 000 aufweisen.

11. Emulsion nach einem der Ansprüche 9 bis 10, wobei die Polyalkylpolyethersiloxane der Formel (V) entsprechen, worin die Gruppen R₇ und R₉ identisch sind und Methylgruppen bedeuten und die Gruppe R₁₀ Wasserstoff bedeutet.

12. Emulsion nach einem der Ansprüche 9 bis 10, wobei die Polyalkylpolyethersiloxane der Formel (V) entsprechen, worin die Gruppen R₇ Methylgruppen bedeuten und die Gruppen R₉ Laurylgruppen bedeuten.

13. Emulsion nach einem der Ansprüche 1 bis 12, wobei die Polyalkylpolyethersiloxane in einer Wirkstoffmenge von 0,2 bis 5 Gew.-% und vorzugsweise 0,25 bis 3 Gew.-%, bezogen auf das Gesamtgewicht der Zusammensetzung, vorliegen.

14. Emulsion nach einem der Ansprüche 1 bis 13, die ferner mindestens eine wasserlösliches oder hydrophiles Verdickungsmittel und/oder einen wasserlöslichen oder hydrophilen Gelbildner enthalten.

15. Emulsion nach einem der Ansprüche 1 bis 14, **dadurch gekennzeichnet, dass** sie weniger als 0,5 Gew.-% eines herkömmlichen grenzflächenaktiven Emulgators enthält.

16. Emulsion nach einem der Ansprüche 1 bis 15, **dadurch gekennzeichnet, dass** sie keinen herkömmlichen grenzflächenaktiven Emulgator enthält.

17. Emulsion nach Anspruch 14, wobei das Verdickungsmittel und/oder der Gelbildner ausgewählt ist unter:
- Algenextrakten;
- Extrakten aus Samen;
- Pflanzenexsudaten;
- Exsudaten aus Mikroorganismen;
- Cellulose und ihren Derivaten und modifizierten Cellulosen;
- Fruchtextrakten;
- Gelbildnern tierischer Herkunft;
- synthetischen Polymeren;
- Siliciumderivaten;
- und den Gemischen dieser Verbindungen.

18. Emulsion nach Anspruch 17, wobei die synthetischen verdickenden und/oder gelierenden Polymere ausgewählt sind unter:
a) den Homopolymeren von Acrylsäure, die mit einem Allylether eines Alkohol aus der Zuckerserie vernetzt sind;
b) vernetzten Copolymeren, die hauptsächlich Acrylsäure und eine geringeren Anteil eines C₁₀₋₃₀-(Meth)acrylsäureesters enthalten;
c) vernetzten Homopolymeren von 2-Acrylamido-2-methylpropansulfonsäure und ihren vernetzten Acrylamid-Copolymeren, die ganz oder zum Teil neutralisiert sind;
d) Homopolymeren von Ammoniumacrylat oder Copolymeren von Ammoniumacrylat und Acrylamid;
e) Homopolymeren von Dimethylaminoethylmethacrylat, das mit Methylchlorid quaternisiert ist, oder Copolymeren von Dimethylaminoethylmethacrylat, das mit Methylchlorid quaternisiert ist, und Acrylamid.

19. Emulsion nach einem der Ansprüche 14 bis 18, wobei die Verdickungsmittel und/oder Gelbildner in Konzentrationen von 0,1 bis 10 Gew.-% und insbesondere 0,1 bis 5 Gew.-%, bezogen auf das Gesamtgewicht der Zusammensetzung, verwendet werden.

20. Verwendung einer Öl-in-Wasser-Emulsion nach einem der Ansprüche 1 bis 19 als oder zur Herstellung von kosmetischen und/oder dermatologischen Zusammensetzungen, die zum Schutz der Haut und/oder der Haare und anderer menschlicher Keratinsubstanzen gegen UV-Strahlung und insbesondere gegen Sonnenlicht vorgesehen sind.

21. Kosmetische und/oder dermatologische Zusammensetzung, **dadurch gekennzeichnet, dass** sie mindestens eine Öl-in-Wasser-Emulsion nach einem der Ansprüche 1 bis 19 enthält.

22. Zusammensetzung nach Anspruch 21, die ferner ein oder mehrere im UV-A- und/oder UV-B-Bereich wirksame, hydrophile oder lipophile Sonnenschutzfilter enthält.

23. Zusammensetzung nach Anspruch 22, wobei die zusätzlichen Filter unter den Zimtsäurederivaten, Salicylsäurederivaten, Dibenzoylmethanderivaten, Benzylidencampherderivaten, Benzimidazolderivaten, Benzophenonderivaten, β,β-Diphenylacrylatderivaten, p-Aminobenzoesäurederivaten und polymeren Filtern und Siliconfiltern ausgewählt sind.

24. Zusammensetzung nach Anspruch 22 oder 23, wobei es sich bei dem zusätzlichen Filter um die Benzol-1,4-di(3-methyliden-10-camphersulfonsäure) und/oder die 2-Phenyl-benzimidazol-5-sulfonsäure handelt.

25. Zusammensetzung nach Anspruch 22 oder 23, wobei es sich bei dem zusätzlichen Filter um das 4-t-Butyl-4'-methoxy-dibenzoylmethan und/oder das 2-Ethylhexyl-α-cyano-β,β'-diphenylacrylat handelt.

26. Zusammensetzung nach einem der Ansprüche 21 bis 24, die ferner mindestens ein umhülltes oder nicht umhülltes Metalloxid-Pigment oder Metalloxid-Nanopigment enthält.

27. Zusammensetzung nach einem der Ansprüche 21 bis 24, die ferner mindestens ein Mittel zur Bräunung und/oder künstlichen Braunfärbung der Haut enthält.

28. Verfahren zur kosmetischen Behandlung zum Schutz der Haut und/oder der Haare und anderer menschlicher Keratinsubstanzen gegen UV-Strahlung und insbesondere Sonnenlicht, das im Wesentlichen darin besteht, auf diese eine Öl-in-Wasser-Emulsion nach einem der Ansprüche 1 bis 20 oder eine Zusammensetzung nach einem der Ansprüche 21 bis 27 in einer wirksamen Menge aufzutragen.

29. Verwendung von Triazinderivaten nach einem der Ansprüche 1 bis 6 als Stabilisierungsmittel für die Herstellung von Öl-in-Wasser-Emulsionen, die mindestens ein Polyalkylpolyethersiloxan nach einem der Ansprüche 1, 8 bis 12 enthalten, das auf die Siliconkette gepfropfte polyalkoxylierte Gruppen aufweist.

## Claims

1. Oil-in-water emulsion, **characterized in that** it comprises at least:
i) one 1,3,5-triazine derivative of following formula: 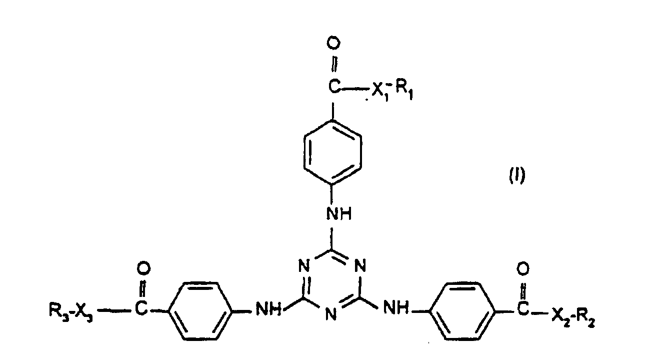 in which:
- X₁, X₂ and X₃, which are identical or different, represent oxygen or an -NR- radical;
- the R₁ radicals, which are identical or different, denote hydrogen or a linear or branched C₁-C₁₈ alkyl radical or a C₅-C₁₂ cycloalkyl radical which can be substituted by one or more C₁-C₄ alkyl radicals;
- R₁, R₂ and R₃, which are identical or different, are chosen from: hydrogen; an alkali metal; an ammonium radical optionally substituted by one or more alkyl or hydroxyalkyl radicals; a linear or branched C₁-C₁₈ alkyl radical; a C₅-C₁₂ cycloalkyl radical optionally substituted by one or more C₁-C₄ alkyl radicals; a polyoxyethylenated radical comprising from 1 to 6 ethylene oxide units, the terminal OH group of which is methylated; or a radical of following formula (II), (III) or (IV): 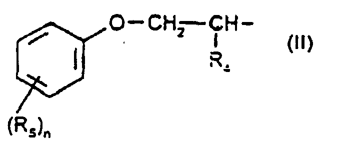 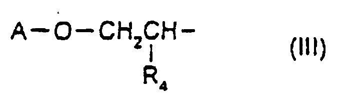 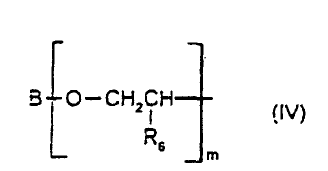 in which formulae:
- R₄ is hydrogen or a methyl radical;
- R₅ is a C₁-C₉ alkyl radical;
- n is an integer ranging from 0 to 3;
- m is an integer ranging from 1 to 10;
- A is a C₄-C₈ alkyl radical or a C₅-C₈ cycloallcyl radical;
- B is chosen from: a linear or branched C₁-C₈ alkyl radical; a C₅-C₈ cycloalkyl radical; or an aryl radical optionally substituted by one or more C₁-C₄ alkyl radicals;
- R₆ is hydrogen or a methyl radical;
ii) one polyalkylpolyethersiloxane carrying polyoxyalkylenated groups grafted onto the main silicone chain; with the proviso that the said emulsion does not comprise cetylstearyltrimethylammonium chloride.

2. Emulsion according to Claim 1, where the 1,3,5-triazine derivatives of formula (I) exhibit all of the following characteristics:
- X₁, X₂ and X₃ are identical and represent oxygen;
- R₁ is chosen from: a C₅-C₁₂ cycloalkyl radical optionally substituted by one or more C₁-C₄ alkyl radicals; or a radical of above formula (II), (III) or (IV), in which formulae:
- B is a C₁-C₄ alkyl radical;
- R₆ is the methyl radical;
- R₂ and R₃, which are identical or different, are chosen from: hydrogen; an alkali metal; an ammonium radical optionally substituted by one or more alkyl or hydroxyalkyl radicals; a linear or branched C₁-C₁₈ alkyl radical; a C₅-C₁₂ cycloalkyl radical optionally substituted by one or more C₁-C₄ alkyl radicals; or a radical of above formula (II), (III) or (IV), in which formulae:
- B is a C₁-C₄ alkyl radical;
- R₆ is the methyl radical.

3. Emulsion according to Claim 1, where the 1,3,5-triazine derivatives of formula (I) exhibit all of the following characteristics:
- X₁ is oxygen; X₂ is the NH radical or oxygen;
- X₃ is the -NH- radical;
- R₃ is chosen from: a linear or branched C₁-C₁₈ alkyl radical; or a C₅-C₁₂ cycloalkyl radical optionally substituted with one or more C₁-C₄ alkyl radicals;
- R₁ is chosen from: hydrogen; an alkali metal; an ammonium radical; a radical of formula (IV); a linear or branched C₁-C₁₈ alkyl radical; or a C₅-C₁₂ cycloalkyl radical optionally substituted with one or more C₁-C₄ alkyl radicals;
- if X₂ is the -NH- radical, then R₂ is chosen from: a linear or branched C₁-C₁₈ alkyl radical; or a C₅-C₁₂ cycloalkyl radical optionally substituted with one or more C₁-C₄ alkyl radicals;
- if X₂ is oxygen, then R₂ is chosen from hydrogen; an alkali metal; an ammonium radical; a radical of formula (IV); a linear or branched C₁-C₁₈ alkyl radical; or a C₅-C₁₂ cycloalkyl radical optionally substituted with one or more C₁-C₄ alkyl radicals.

4. Emulsion according to Claim 1, where the 1,3,5-triazine derivatives of formula (I) exhibit all of the following characteristics:
- X₁, X₂ and X₃ simultaneously denote -NR-;
- the R radicals, which are identical or different, denote hydrogen or a linear or branched C₁-C₁₈ alkyl radical or a C₅-C₁₂ cycloalkyl radical which can be substituted by one or more C₁-C₄ alkyl radicals;
- R₁, R₂ and R₃, which are identical or different, denote hydrogen or an R radical.

5. Emulsion according to Claim 1, where the 1,3,5-triazine derivative corresponds to the following formula: 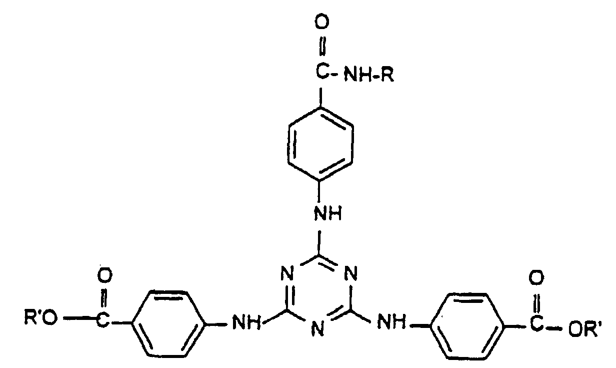 in which R' denotes a 2-ethylhexyl radical and R denotes a tert-butyl radical.

6. Emulsion according to Claim 1, where the 1,3,5-triazine derivative corresponds to the following formula: 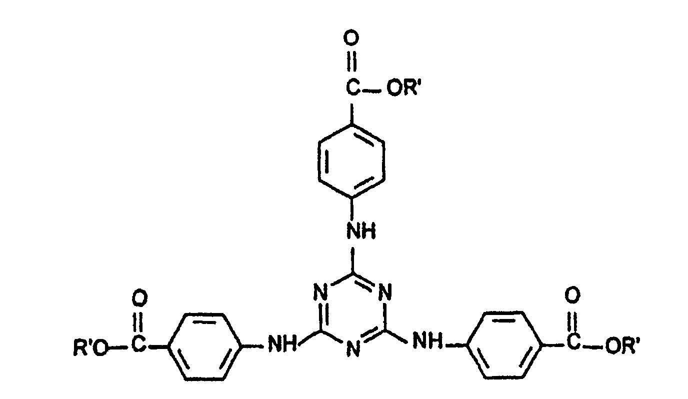 in which R' denotes a 2-ethylhexyl radical.

7. Emulsion according to any one of Claims 1 to 6, where the 1,3,5-triazine derivatives are present at a content ranging from 0.5% to 20%, preferably from 1% to 10%, by weight with respect to the total weight of the composition.

8. Emulsion according to any one of Claims 1 to 7, where the polyalkylpolyethersiloxanes comprise polyoxyethylene and/or polyoxypropylene chains grafted onto the main chain.

9. Emulsion according to any one of Claims 1 to 7, where the polyalkylpolyethersiloxanes are chosen from the compounds of following general formula (V): 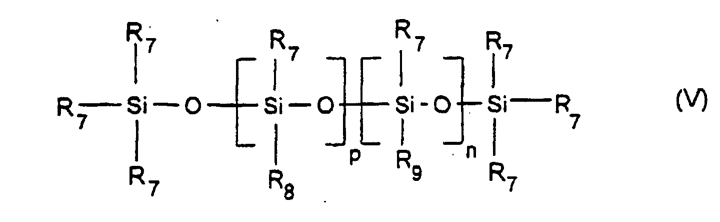 in which formula:
- R₇ and R₉, which are identical or different, represent a hydrogen atom, a linear or branched C₁-C₃₀ alkyl radical or a phenyl radical,
- R₈, which are identical or different, represent - (CₓH₂ₓ)-(OC₂H₄)ₐ-(OC₃H₆)_{b}-OR₁₀,
- R₁₀, which are identical or different, are chosen from a hydrogen atom, a linear or branched alkyl radical having from 1 to 12 carbon atoms or a linear or branched acyl radical having from 2 to 12 carbon atoms,
- n varies from 1 to 1000,
- p varies from 1 to 30,
- a varies from 1 to 50,
- b varies from 0 to 50,
- x varies from 1 to 5.

10. Emulsion according to any one of Claims 1 to 7, where the polyalkylpolyethersiloxanes exhibit a number-average molecular weight of greater than or equal to 15,000 and preferably ranging from 20,000 to 40,000.

11. Emulsion according to either one of Claims 9 and 10, where the polyalkylpolyethersiloxanes correspond to the formula (V) in which the R₇ and R₉ radicals are identical and all represent methyl radicals and the R₁₀ radical represents hydrogen.

12. Emulsion according to either one of Claims 9 and 10, where the polyalkylpolyethersiloxanes correspond to the formula (V) in which the R₇ radicals all represent methyl radicals and the R₉ radicals all represent lauryl radicals.

13. Emulsion according to any one of Claims 1 to 12, where the polyalkylpolyethersiloxanes are present in a proportion of active material ranging from 0.2% to 5% by weight, preferably from 0.25% to 3% by weight, with respect to the total weight of the composition.

14. Emulsion according to any one of Claims 1 to 13, additionally comprising at least one water-soluble or hydrophilic thickening and/or gelling agent.

15. Emulsion according to any one of Claims 1 to 14, **characterized in that** it comprises less than 0.5%. by weight of conventional emulsifying surface-active agent.

16. Emulsion according to any one of Claims 1 to 15, **characterized in that** it does not comprise a conventional emulsifying surface-active agent.

17. Emulsion according to Claim 14, where the thickening and/or gelling agent is chosen from:
- algal extracts;
- seed extracts;
- plant exudates;
- microorganism exudates;
- cellulose or its derivatives, as well as modified celluloses;
- food extracts;
- gelling agents of animal origin;
- synthetic polymers;
- silicon derivatives,
- mixtures of the above compounds.

18. Emulsion according to Claim 17, where the synthetic thickening and/or gelling polymers are chosen from:
(a) acrylic acid homopolymers crosslinked by an allyl ether of an alcohol of the sugar series;
(b) crosslinked copolymers comprising a major fraction of acrylic acid and a minor fraction of C₁₀-C₃₀ esters of (meth)acrylic acid;
(c) crosslinked homopolymers of 2-acrylamido-2-methylpropanesulphonic acid and their partially or completely neutralized crosslinked copolymers with acrylamide;
(d) ammonium acrylate homopolymers or copolymers of ammonium acrylate and of acrylamide;
(e) homopolymers of dimethylaminoethyl methacrylate quaternized by methyl chloride or copolymers of dimethylaminoethyl methacrylate quaternized by methyl chloride and of acrylamide.

19. Emulsion according to any one of Claims 14 to 18, where the thickening and/or gelling agents are used in concentrations ranging from 0.1 to 10% by weight, in particular from 0.1 to 5% by weight, with respect to the total weight of the composition.

20. Use of an oil-in-water emulsion according to any one of Claims 1 to 9 as, or for the manufacture of, cosmetic or dermatological compositions intended for the protection of the skin and/or hair and other human keratinous substances against ultraviolet radiation, in particular solar radiation.

21. Cosmetic and/or dermatological composition, **characterized in that** it comprises at least one oil-in-water emulsion according to any one of Claims 1-19.

22. Composition according to Claim 21, additionally comprising one or more hydrophilic or lipophilic sunscreen agents active in the UV-A or UV-B.

23. Composition according to Claim 22, where the additional screening agents are chosen from cinnamic derivatives, salicylic derivatives, dibenzoylmethane derivatives, benzylidenecamphor derivatives, benzimidazole derivatives, benzophenone derivatives, β,β-diphenylacrylate derivatives, p-aminobenzoic acid derivatives, or screening polymers and screening silicones.

24. Composition according to Claim 22 or 23, where the additional hydrophilic screening agent is benzene-1,4-di(3-methylidene-10-camphosulphonic acid) and/or 2-phenylbenzimidazole-5-sulphonic acid.

25. Composition according to Claim 22 or 23, where the additional lipophilic screening agent is 4-tert-butyl-4'-methoxydibenzoylmethane and/or 2-ethylhexyl α-cyano-β,β-diphenylacrylate.

26. Composition according to any one of Claims 21 to 24, additionally comprising at least one coated or non-coated metal oxide pigment or nanopigment.

27. Composition according to any one of Claims 21 to 24, additionally comprising at least one agent for the artificial tanning and/or browning of the skin.

28. Cosmetic treatment process for the protection of the skin and/or the hair and/or the other human keratinous substances against ultraviolet radiation, in particular solar radiation, which consists essentially in applying, to the latter, an effective amount of an oil-in-water emulsion as defined according to any one of Claims 1 to 20 or of a composition as defined according to any one of Claims 21 to 27.

29. Use of the 1,3,5-triazine derivatives as defined according to any one of Claims 1 to 6 as stabilizing agent in the preparation of an oil-in-water emulsion comprising at least one polyalkylpolyethersiloxane carrying polyoxyalkylene groups grafted onto the silicone chain as are defined according to any one of Claims 1 and 8 to 12.
